# EUROPEAN PATENT APPLICATION

(11) **EP 3 726 215 A1**
(43) Date of publication of application: **21.10.2020**
(21) Application number: 18889014.9
(22) Date of filing: 11.12.2018
(51) Int. Cl.: G01N 33/543, G01N 33/552, G01N 33/72, G01N 33/68, G01N 33/58, G01N 30/88, G01N 21/31

(54) **SILICA NANOPARTICLES FOR BIOMARKER DIAGNOSIS AND METHOD FOR PRODUCING SAME**

(30) Priority: 12.12.2017 KR 20170170243; 10.12.2018 KR 20180158405
(71) Applicant: DxGen Corp., Gunpo-si, Gyeonggi-do 15807 (KR)
(72) Inventor: LEE, Jin Woo, Suwon-si Gyeonggi-do 16324 (KR); CHEON, Seon Ah, Seoul 06920 (KR)
(74) Representative: Ter Meer Steinmeister & Partner
(86) International application number: PCT/KR2018/015676
(87) International publication number: WO 2019/117586

(57) **Abstract**

The present invention relates to silica nanoparticles for biomarker diagnosis and a method of manufacturing the same, and more particularly to silica nanoparticles for biomarker diagnosis capable of measuring glycated proteins such as glycated hemoglobin, glycated albumin, etc. used as diabetes diagnosis markers, and a method of manufacturing the same. According to the present invention, silica nanoparticles for biomarker diagnosis, containing a reactive polymer and a nonreactive polymer immobilized on the surface and a dye immobilized inside, immobilize the dye therein through covalent bonding, whereby the dye is not affected by external environmental factors such as heat, pH, etc., and stability is enhanced even upon long-term storage. Also, since multiple dye molecules are immobilized in a single silica nanoparticle, the amount of light absorbed by a single particle is increased compared to that of a single dye molecule, making it possible to accurately measure the amount of glycated protein with a low detection limit. Moreover, since the nonreactive polymer for increasing dispersion stability and the probe-bound reactive polymer are immobilized on the surface of the silica nanoparticles containing the dye immobilized inside, the influence of various proteins and salts included in a sample is minimized, whereby the silica nanoparticles can be applied to a point-of-care testing system using an actual biological material to thus quickly and accurately measure a biomarker to be detected in a stable manner.

## Description

### Technical Field

The present invention relates to silica nanoparticles for biomarker diagnosis and a method of manufacturing the same, and more particularly to silica nanoparticles for biomarker diagnosis capable of measuring glycated proteins such as glycated hemoglobin, glycated albumin and the like, used as diabetes diagnosis markers, and a method of manufacturing the same.

### Background Art

Diabetes is a metabolic disease that occurs due to abnormal insulin secretion in blood glucose regulation, and is classified, depending on the causes thereof, into type 1 diabetes, which occurs due to insulin deficiency by destroying insulin-producing cells attributed to abnormality in the immune system, and type 2 diabetes, which occurs when insulin secretion is insufficient or when insulin is not effectively used.

Diabetes is characterized by high blood glucose, in which the concentration of glucose in the blood is high, and failure to regulate blood glucose may lead to complications such as diabetic retinopathy, kidney disease, foot lesions and the like, and thus the importance of managing blood glucose in diabetic patients is increasing.

Recently, glycated hemoglobin (HbA1c) or glycated albumin (GA) has come to be used as a biomarker for measuring blood glucose, and is more accurate and stable than glucose. Glycated hemoglobin, having a stable lifespan, may be used as an indicator that shows the average blood glucose level over 2 to 3 months, and is utilized in practice to investigate the diagnosis and treatment process of diabetes. However, the glycated hemoglobin measurement method is not suitable for patients whose blood glucose is not maintained stable, such as in cases of end-stage chronic renal failure, or for patients with abnormalities in red blood cells.

Albumin is a protein present not only in the blood but also in major organs and body fluids, and becomes glycated albumin bound to glucose depending on the concentration of glucose in the blood. Albumin has a glucose-binding rate about 10 times as high as that of hemoglobin, so glycated albumin may respond more sensitively to changes in blood glucose than glycated hemoglobin. Moreover, albumin, which has a lifespan of about 15 to 20 days compared to red blood cells having a lifespan of about 90 days, is capable of monitoring the average level of glucose in the blood for 2 weeks. Therefore, glycated albumin may be useful as an important blood glucose measurement factor in patients with iron deficiency anemia and diabetic patients with variant hemoglobin, as well as patients with end-stage chronic renal failure in which blood glucose is not maintained stable. With regard to diabetes diagnosis criteria, glycated proteins such as glycated hemoglobin and glycated albumin serve as mutually complementary indicators for reliable diagnosis and patient's health care.

Since the amounts of total hemoglobin and total albumin in persons vary depending on gender, age, blood-related diseases, etc., diabetes diagnosis has to be judged based on the ratio of specific glycated protein relative to the total amount of glycated and non-glycated specific proteins, rather than the absolute content of specific glycated protein. Therefore, in order to use these glycated proteins as diabetes diagnosis markers, a technique capable of distinguishing the amounts of total hemoglobin and total albumin from the amounts of glycated hemoglobin and glycated albumin is required.

In the measurement of glycated hemoglobin, the amount of total hemoglobin is measured using the intrinsic absorption wavelength of hemoglobin, and the amount of glycated hemoglobin is measured in a manner in which boronic acid able to selectively bind to the glycated portion is treated with a material having absorbance or fluorescence properties and then reacted with a sample containing glycated hemoglobin and total hemoglobin, after which the optical properties thereof are analyzed. Hemoglobin is composed of heme, the center of which is coordinated with iron ions, and globin protein, and each hemoglobin has an intrinsic wavelength depending on the chemical modification of hemoglobin such as binding thereof to oxygen, the extent of oxidation, etc., and mostly shows an absorption wavelength in the range of 400 to 600 nm. In particular, the absorption wavelength of 430 nm is a common characteristic of glycated hemoglobin and general hemoglobin, so it may represent the amount of total hemoglobin. The glycated portion is mainly labeled using a boronic-acid affinity method in which boronic acid, which recognizes cis-diol of the glucose site of glycated hemoglobin, is coupled with a coloring dye or a fluorophore and is thus used as a labeling agent, which is advantageous because high sensitivity and stability similar to that of an enzyme reaction or an antigen-antibody reaction.

U.S. Patent No. 5631364, U.S. Patent No. 7374943 and International Patent No. 2014-033258 disclose a method of determining the ratio of two materials by reacting a dye-bound boronic acid derivative with glycated blood hemoglobin, followed by loading in a cartridge made of porous filter paper, washing, and then measurement of reflectance (%) of total hemoglobin and dye-bound glycated hemoglobin.

European Patent Application Publication No. 2444803 and Korean Patent No. 1128037 disclose a method of binding a boronic acid derivative to beads and a method of directly measuring glycated hemoglobin of the reacted sample in a cartridge made of porous filter paper. The synthesis of beads and boronic acid derivative is relatively simple, but for accuracy, careful attention must be paid at various steps of measuring hemoglobin and glycated hemoglobin. This is because total hemoglobin is first measured at 430 nm, normal hemoglobin is removed by washing, and then glycated hemoglobin remaining after reaction with the bead/boronic-acid derivative is measured at the same wavelength.

As a conventional technique of measuring glycated albumin, there is an enzymatic method. U.S. Patent No. 7871789, U.S. Patent No. 6008006, U.S. Patent No. 8507223, Chinese Patent Application Publication No. 104673878 and Chinese Patent Application Publication No. 104614459 disclose a conventional method of measuring glycated albumin, specifically a glycated-albumin enzymatic method, in which glycated protein present in a sample is decomposed into glycated amino acid or glycated peptide using protease, followed by measurement of hydrogen peroxide (H₂O₂), produced by the enzyme reaction of glycated amino acid oxidase (EC 1.5.3) series that specifically reacts therewith, using a peroxidase enzyme.

The glycated-albumin enzymatic method exhibits high selectivity and accuracy for albumin and also enables testing within 10 min to 30 min, faster than the immunoassay method. However, proteins such as enzymes have activity varying depending on the external environment or reaction conditions, and moreover, the activity thereof has a great influence on glycated-albumin analysis efficiency, so strict attention is required in order to maintain the activity of the enzyme and when storing the enzyme.

The glycated-albumin enzymatic method is a complicated measurement method composed of multiple steps because it is necessary to first perform a step of removing glycated amino acid and glycated peptide already present in the sample and also because glycated amino acid oxidase (EC 1.5.3) cannot use intact glycated protein as a substrate, and thus a step of decomposing glycated protein into glycated amino acid or peptide using protease must be performed first. Hence, there is a need to develop a method that is able to more simply and quickly measure glycated albumin.

U.S. Patent No. 5223392, U.S. Patent No. 5908925, European Patent Application Publication No. 0657470, European Patent No. 0257421 and Chinese Patent Application Publication No. 103554256 disclose an enzyme immunoassay (ELISA) using an albumin antibody and a glycated albumin antibody immobilized with a peroxidase enzyme in order to detect glycated albumin. Moreover, Ikeda *et al.* reported an enzyme/boronic-acid immunoassay (ELIBA) using an aluminum antibody and boronic acid immobilized with a peroxidase enzyme (Ikeda et al., Clin Chem. 44(2):256-263, 1998). These methods have high selectivity and accuracy, but require storage and maintenance of antibodies and enzymes and take a lot of time (30 min to 90 min) to perform a test, so limitations are imposed on development of onsite diagnosis and high-speed detection kits.

As for onsite diagnosis and high-speed detection kits, U.S. Patent No. 9128085, U.S. Patent Application Publication No. 2006-0270060, U.S. Patent Application Publication No. 2008-0227210, U.S. Patent Application Publication No. 2010-0167306, U.S. Patent No. 5470759 and U.S. Patent No. 7659107 disclose a disposable strip and cassette using antibody-based lateral flow immunochromatography in order to measure albumin and glycated albumin in samples such as blood, saliva, and the like, U.S. Patent Application Publication No. 2014-0170766 discloses a method of manufacturing a rapid kit through lateral flow immunochromatography using a nucleic-acid-derived albumin aptamer and a glycated-albumin aptamer, which have action similar to antibodies, and U.S. Patent Application Publication No. 2014-0335630 discloses an assay method using a glycated-albumin aptamer and SPR (surface plasmon resonance). However, attention must be paid to maintenance and storage of the aptamer, composed of nucleic acid, as in the antibody. Therefore, it is necessary to develop a diagnosis reagent and a measurement method capable of more stably and quickly measuring glycated albumin.

Although research on rapid diagnosis kits for glycated hemoglobin using a boronic-acid affinity method has been reported, rapid diagnosis kits for glycated albumin using a boronic-acid affinity method have not yet been reported. This is because there are limitations in application of the boronic-acid affinity method to glycated-albumin measurement. Specifically, xylene-cyanol/DAPOL/CPBA, which is a "dye/boronic-acid derivative" mainly used for the measurement of glycated hemoglobin, has an absorption wavelength of 620 nm, which is the same as that of bromocresol green or bromocresol purple used for albumin measurement, and is thus unsuitable for the measurement of glycated albumin. For the measurement of glycated albumin, it is necessary to develop a boronic acid derivative coupled with a dye other than bromocresol green or purple.

In order implement onsite diagnosis technology for measurement of a biomarker such as glycated hemoglobin or glycated albumin, an actual biological material from a patient has to be reacted with the boronic acid derivative coupled with the dye mentioned above. However, the organic-based dye-immobilized boronic acid derivative is problematic because the corresponding material is exposed to the external environment and is thus vulnerable to light and heat and also because the synthesis process thereof is complicated. Moreover, there are required methods for minimizing problems in which the amount of color development and the wavelength of color development vary depending on surrounding environmental factors such as the pH of the reaction system, polarity, other impurities and the like, or in which light is sometimes extinguished.

Therefore, the present inventors have made great efforts to solve the problems with existing detection methods and the demand for improved detection technology, and have ascertained that the stability and the extent of color development of a dye may be improved when using nanoparticles containing a dye immobilized inside through covalent bonding, rather than simply using the dye as is.

In this case, however, there is a problem in which the stability of the nanoparticles may deteriorate upon reaction thereof with a non-pretreated biological material. Hence, the present inventors have made further efforts to solve the above problem, and have ascertained that, when silica nanoparticles containing a dye immobilized inside are treated with a nonreactive polymer for improving dispersion stability and a probe-bound reactive polymer, the stability of the silica nanoparticles in a buffer solution and in an actual sample may be greatly improved, and the reaction of the silica nanoparticles and the target material may be efficiently carried out even upon reaction with the actual sample, thus culminating in the present invention.

### Disclosure

### Technical Problem

An objective of the present invention is to provide, as a platform technology to quickly and accurately measure diseases that require periodic diagnosis, such as diabetes, through point-of-care testing, the synthesis of a marker material, in which a dye inside silica nanoparticles is selectively immobilized depending on the properties of each target material, and which is treated with a nonreactive polymer for increasing dispersion stability even in environments with various proteins or salts contained in a sample due to the properties of a biological material and with a reactive polymer coupled with a material able to selectively recognize the target material.

Another objective of the present invention is to provide a diagnosis method of quickly and accurately measuring glycated hemoglobin and glycated albumin, which are biomarkers for diabetes diagnosis, by mixing the inventive material with a reaction buffer solution.

### Technical Solution

In order to accomplish the above objectives, the present invention provides silica nanoparticles for biomarker diagnosis containing a reactive polymer and a nonreactive polymer immobilized on the surface and a dye immobilized inside.

In addition, the present invention provides a method of manufacturing silica nanoparticles for biomarker diagnosis containing a reactive polymer and a nonreactive polymer immobilized on the surface and a dye immobilized inside, including (a) reacting a dye and a silica precursor, (b) manufacturing "silica nanoparticles containing a dye immobilized inside" by adding the reaction product of the dye and the silica precursor and silicate to a mixed solution of a surfactant and an organic solvent, performing stirring and then adding a basic catalyst thereto, (c) immobilizing a "reactive polymer" and a "nonreactive polymer" on the surface of the "silica nanoparticles containing a dye immobilized inside" by stirring the "reactive polymer" and the "nonreactive polymer" with the "silica nanoparticles containing a dye immobilized inside", and (d) binding a desired probe to the "reactive polymer".

In addition, the present invention provides a method of diagnosing diabetes, including measuring optical reflectance by simultaneously applying a wavelength able to measure total albumin or total hemoglobin and a wavelength able to measure glycated albumin or glycated hemoglobin bound to "silica nanoparticles for biomarker diagnosis containing a reactive polymer and a nonreactive polymer immobilized on the surface and a dye immobilized inside / boronic acid" using a light source, and then diagnosing diabetes depending on the proportion of glycated albumin or glycated hemoglobin.

In addition, the present invention provides a method of diagnosing diabetes, including reacting a biological material with "silica nanoparticles for biomarker diagnosis containing a reactive polymer and a nonreactive polymer immobilized on the surface and a dye immobilized inside / boronic acid", performing high-performance liquid chromatography (HPLC), and then diagnosing diabetes depending on the proportion of glycated albumin or glycated hemoglobin.

### Advantageous Effects

According to the present invention, silica nanoparticles for biomarker diagnosis, containing a reactive polymer and a nonreactive polymer immobilized on the surface and a dye immobilized inside, immobilize the dye therein through covalent bonding, whereby the dye is not affected by external environmental factors such as heat, pH, and the like, and stability can be increased even upon long-term storage, and moreover, multiple dye molecules are immobilized in a single silica nanoparticle, whereby the amount of light absorbed by a single particle is increased compared to the absorption amount of a single dye molecule, making it possible to accurately measure the amount of glycated protein with a low detection limit.

### Brief Description of Drawings

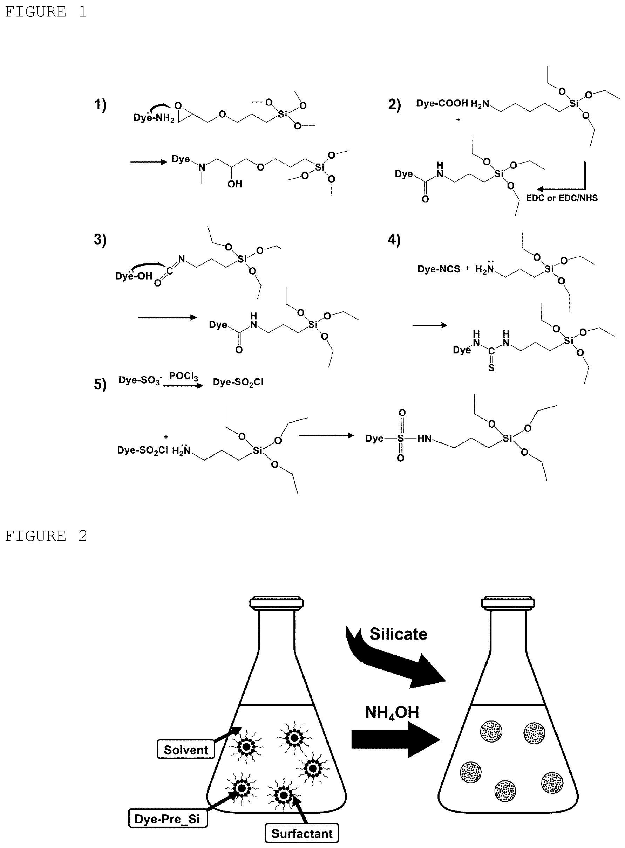

FIG. 1 shows the reaction mechanism for covalent bonding of a dye and a silica precursor (Pre_Si);
FIG. 2 shows a process of manufacturing "silica nanoparticles containing a dye immobilized inside" according to an embodiment of the present invention;

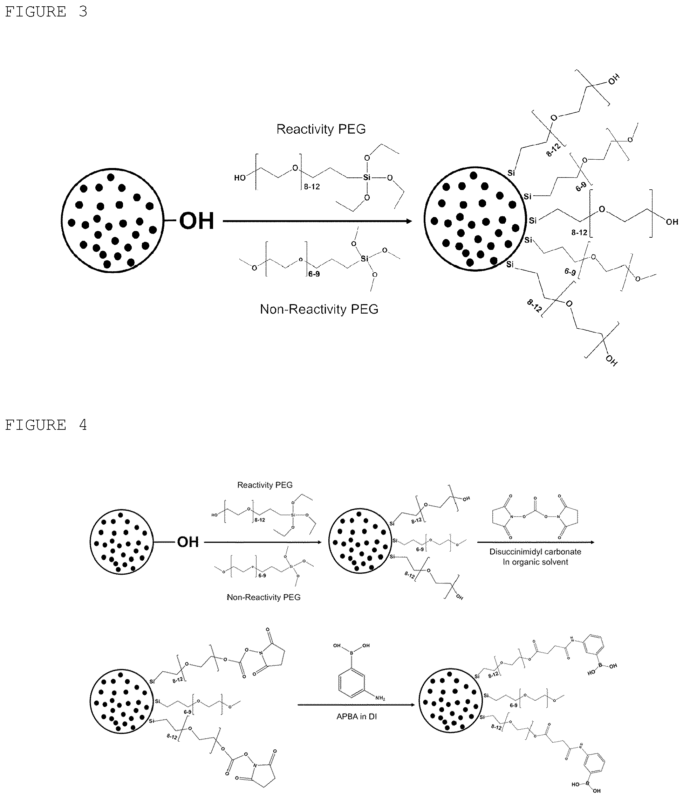

FIG. 3 shows a process of synthesizing "silica nanoparticles for biomarker diagnosis containing a reactive polymer and a nonreactive polymer immobilized on the surface and a dye immobilized inside" by immobilizing a reactive polymer and a nonreactive polymer on the surface of the "silica nanoparticles containing a dye immobilized inside" according to an embodiment of the present invention;
FIG. 4 shows a process of manufacturing 3-aminophenylboronic-acid-(APBA)-bound "silica nanoparticles for biomarker diagnosis containing a reactive polymer and a nonreactive polymer immobilized on the surface and a dye immobilized inside" according to an embodiment of the present invention;

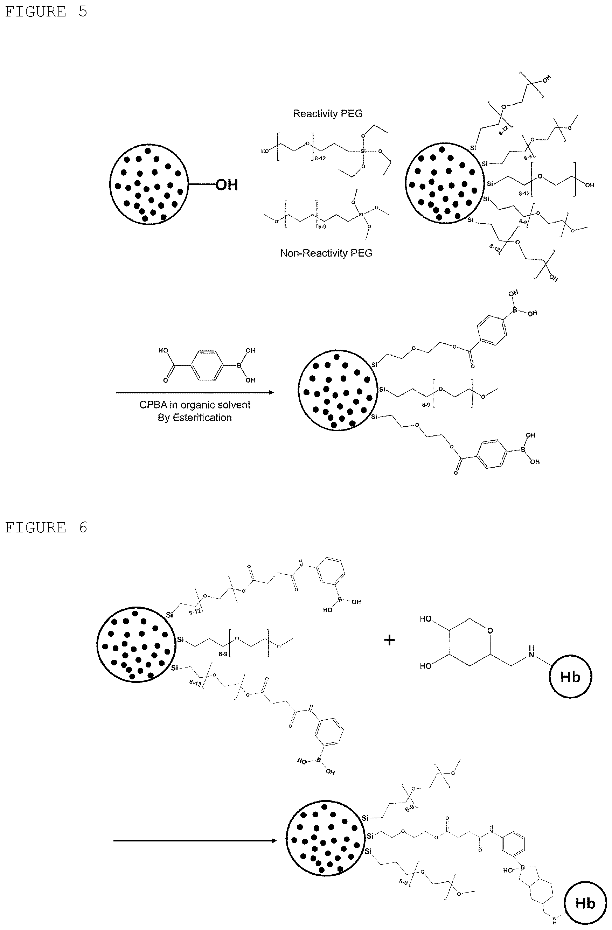

FIG. 5 shows a process of manufacturing 4-carboxylphenylboronic-acid-(CBPA)-bound "silica nanoparticles for biomarker diagnosis containing a reactive polymer and a nonreactive polymer immobilized on the surface and a dye immobilized inside" according to an embodiment of the present invention;
FIG. 6 shows the cis-diol bond of glycated hemoglobin and the "silica nanoparticles for biomarker diagnosis containing a reactive polymer and a nonreactive polymer immobilized on the surface and a dye immobilized inside" manufactured according to an embodiment of the present invention;

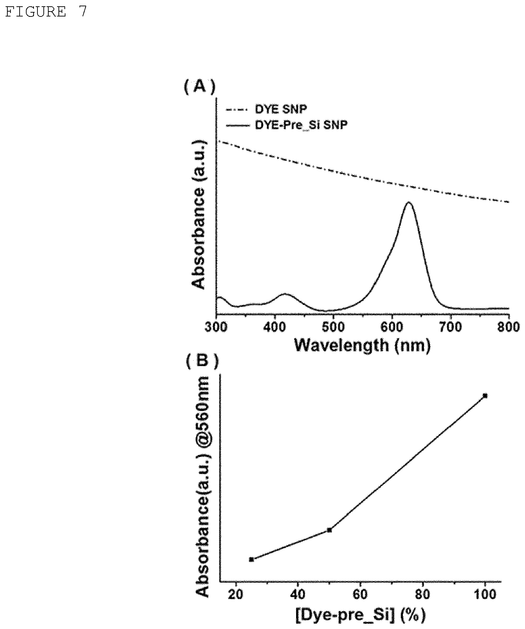

FIG. 7 is graphs showing the results of measurement of absorbance of the "silica nanoparticles for biomarker diagnosis containing a reactive polymer and a nonreactive polymer immobilized on the surface and a dye immobilized inside (pDSNP-APBA)" manufactured according to an embodiment of the present invention (A: comparison of absorbance spectrum thereof with nanoparticles containing a dye encapsulated alone (DYE SNP), B: increased absorbance of pDSNP-APBA depending on the concentration of Dye-Pre_Si used for synthesis);

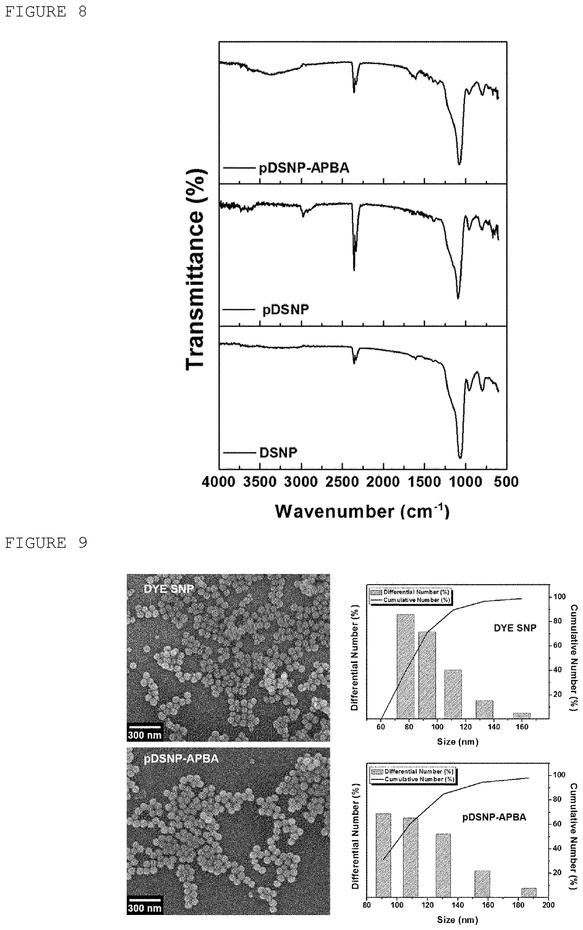

FIG. 8 shows FT-IR graphs of the "silica nanoparticles containing a dye immobilized inside (DSNP)", "silica nanoparticles containing a reactive polymer and a nonreactive polymer immobilized on the surface and a dye immobilized inside (pDSNP)", and "silica nanoparticles for biomarker diagnosis containing a reactive polymer and a nonreactive polymer immobilized on the surface and a dye immobilized inside / boronic acid (pDSNP-APBA)", manufactured according to an embodiment of the present invention;
FIG. 9 shows the shapes and size distribution graphs of the "silica nanoparticles for biomarker diagnosis containing a reactive polymer and a nonreactive polymer immobilized on the surface and a dye immobilized inside" manufactured according to an embodiment of the present invention and silica nanoparticles containing a dye encapsulated alone as a comparative example;

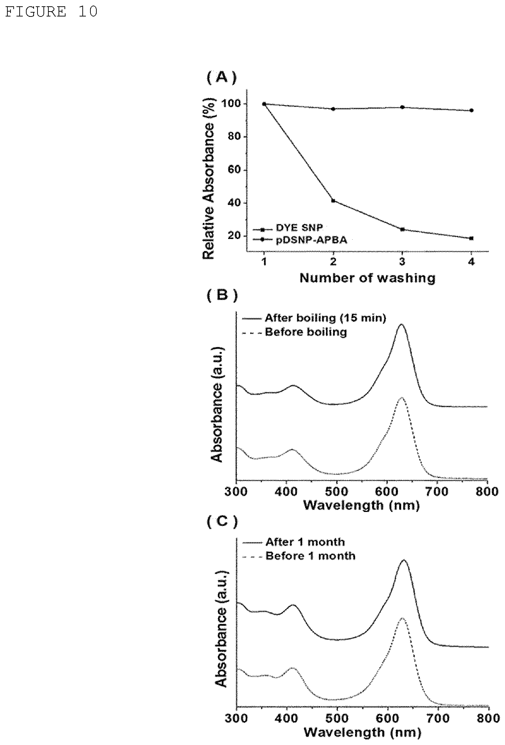

FIG. 10 is graphs showing the results of measurement of absorbance after treatment under harsh conditions of the "silica nanoparticles for biomarker diagnosis containing a reactive polymer and a nonreactive polymer immobilized on the surface and a dye immobilized inside" manufactured according to an embodiment of the present invention (A: repeated washing, B: heating at 100°C for 15 min, C: storage at room temperature for 30 days);

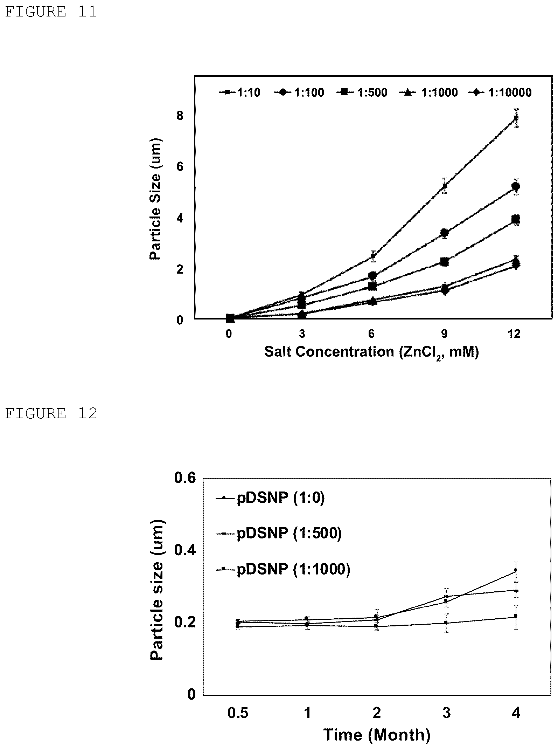

FIG. 11 is a graph showing the results of evaluation of dispersion stability depending on the salt concentration under the condition of increasing the amount of the polymer immobilized to the "silica nanoparticles for biomarker diagnosis containing a reactive polymer and a nonreactive polymer immobilized on the surface and a dye immobilized inside" manufactured according to an embodiment of the present invention;
FIG. 12 is a graph showing the results of evaluation of long-term dispersion stability depending on the ratio of a reactive polymer to a nonreactive polymer of the "silica nanoparticles for biomarker diagnosis containing a reactive polymer and a nonreactive polymer immobilized on the surface and a dye immobilized inside" manufactured according to an embodiment of the present invention;

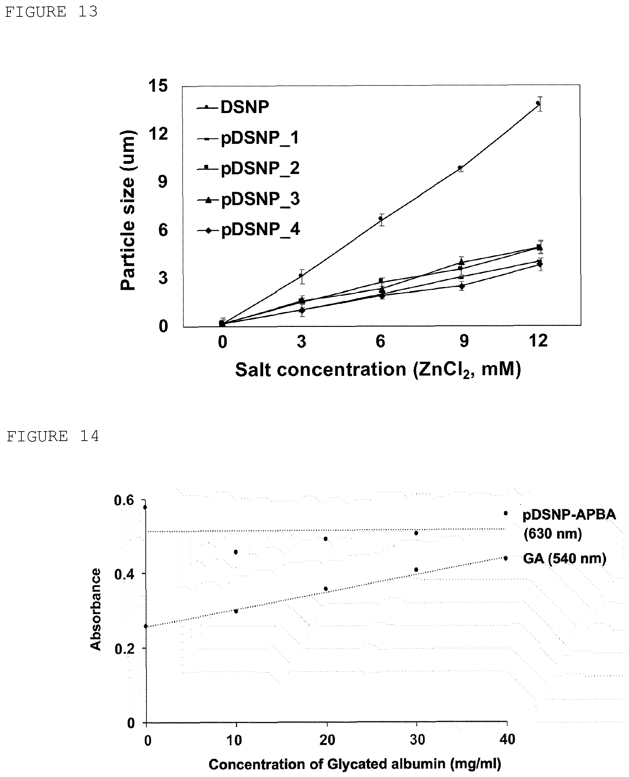

FIG. 13 is a graph showing the reduction in agglomeration depending on the salt concentration of the "silica nanoparticles for biomarker diagnosis containing a reactive polymer and a nonreactive polymer immobilized on the surface and a dye immobilized inside" manufactured according to an embodiment of the present invention; and
FIG. 14 is a graph showing the results of evaluation of binding capacity depending on the concentration of glycated albumin and the "silica nanoparticles for biomarker diagnosis containing a reactive polymer and a nonreactive polymer immobilized on the surface and a dye immobilized inside" manufactured according to an embodiment of the present invention.

### Best Mode

The present invention is intended to quickly and accurately measure a biomarker to be detected in a stable manner using silica nanoparticles for biomarker diagnosis containing a dye having various absorption wavelengths immobilized inside and a nonreactive polymer for improving the dispersion stability of particles and a reactive polymer coupled with a probe specifically binding to a biomarker immobilized on the surface thereof.

In the present invention, a dye having a functional group enabling mutual covalent bonding and a silica precursor are reacted together and then reacted with silica in a solvent, thus manufacturing "silica nanoparticles containing a dye immobilized inside", after which a nonreactive polymer for increasing the dispersion stability of particles in a biological material and a reactive polymer having a functional group able to bind to a biomarker to be labeled are immobilized on the surface of the silica nanoparticles, thereby manufacturing "silica nanoparticles for biomarker diagnosis containing a reactive polymer and a nonreactive polymer immobilized on the surface and a dye immobilized inside".

Even when the "silica nanoparticles for biomarker diagnosis containing a reactive polymer and a nonreactive polymer immobilized on the surface and a dye immobilized inside" thus manufactured are stored at room temperature for 30 days or exposed at 100°C for 15 min or repetitively washed, it is confirmed that the intrinsic wavelength of the dye is maintained without change and the dispersion stability of the silica nanoparticles is high not only in a buffer solution such as HEPES, etc. but also in an actual biological material.

Therefore, the present invention pertains to "silica nanoparticles for biomarker diagnosis containing a reactive polymer and a nonreactive polymer immobilized on the surface and a dye immobilized inside" and a method of manufacturing the same.

The method of manufacturing the "silica nanoparticles for biomarker diagnosis containing a reactive polymer and a nonreactive polymer immobilized on the surface and a dye immobilized inside" according to the present invention includes (a) reacting a dye and a silica precursor, (b) manufacturing "silica nanoparticles containing a dye immobilized inside" by adding the reaction product of the dye and the silica precursor and silicate to a mixed solution of a surfactant and an organic solvent, performing stirring and then adding a basic catalyst thereto, (c) immobilizing a "reactive polymer" and a "nonreactive polymer" on the surface of the "silica nanoparticles containing a dye immobilized inside" by stirring the "reactive polymer" and the "nonreactive polymer" with the "silica nanoparticles containing a dye immobilized inside", and (d) binding a desired probe to the "reactive polymer".

In the present invention, the biomarker may be exemplified by a blood protein, including glycated hemoglobin or glycated albumin, but is not limited thereto.

In the present invention, the dye is used to measure the biomarker, and may include organic or fluorescent dyes having various spectra. In order to form a covalent bond with silica nanoparticles, a dye having a functional group such as hydroxyl (-OH), carboxyl (-COOH), amine (-NH or -NH₂), isothiocyanate (-NCO), sulfonate (-SO₃), etc. is preferably used.

Examples of the dye having a hydroxyl functional group may include, but are not limited to, FD&C Green 3, calcein blue, calcein blue AM, Carmine, Coelenterazine, Coelenterazine cp, Coelenterazine f, Coelenterazine h, Coelenterazine hcp, Coelenterazine n, CoroNa Green, Hematoxylin, Hoechst 33258, Newport green pdx, Oil red O, Orange II, Orcein, Oxonol V, Oxonol VI, Ponceau SX, Sudan III, Sudan IV, Sunset Yellow FCF, and the like.

Also, examples of the dye having a carboxyl functional group may include, but are not limited to, tartrazine, betanin, bixin, carminic acid, galiosin, pseudo-purpurin, BAPTA, BTC (benzene-1,3,5-tricarboxylic acid), calcein, calcium green 1, calcium green 2, calcium green 5N, Eosin B, Eosin Y, erythrosine, Fluo-3, Fluo-4, FluoZin-2, FluoZin-3, Fura-2, Fura-2FF, Indo 1, PBFI (potassium-binding benzofuran isophthalate), RhodZin-3, SBFI (sodium benzofuran isophthalate), Zinquin, and the like.

Also, examples of the dye having a primary amine functional group may include, but are not limited to, ACMA (9-amino-6-chloro-2-methoxyacridine), methylene blue, Nile blue, toluidine blue O, Azure A, Azure B, basic fuchsin, Bismarck brown Y, brilliant cresyl blue, cresyl violet acetate, DAF-FM (diaminofluorescein-FM), dansylcadaverine, DAPI (4',6-diamidino-2-phenylindole), dihydroethidium, dihydrorhodamine 123, ethidium bromide, ethidium homodimer-1, ethidium homodimer-2, ethidium monoazide, hexidium iodide, Lucifer yellow CH, Lucifer yellow VS, neutral red, nuclear yellow, pararosaniline hydrochloride, propidium iodide, rhodamine 123, Safranin O, thionine, and the like, and the dye having a secondary amine functional group may be exemplified by Auramine O, but is not limited thereto.

Also, examples of the dye having an isothiocyanate functional group may include, but are not limited to, fluorescein isothiocyanate, malachite green isothiocyanate, rhodamine B isothiocyanate, stilbene isothiocyanate sulfonic acid and the like.

Also, examples of the dye having a sulfonate functional group may include, but are not limited to, xylene cyanol FF, Alizarin red S, Allura red, amaranth, aniline blue, brilliant blue FCF, Congo red, Indigo carmine, Light Green SF Yellowish, methyl orange, Orange G, SPQ (6-methoxy-N-(3-sulfopropyl)quinolinium), XTT (2,3-bis-(2-methoxy-4-nitro-5-sulfophenyl)-2H-tetrazolium-5-carboxanilide), and the like.

FIG. 1 shows the reaction mechanism for covalent bonding of a dye and a silica precursor (Pre Si). In order to manufacture "silica nanoparticles containing a dye immobilized inside", a dye and a silica precursor are first reacted with stirring. Here, the silica precursor preferably includes 3-aminopropyltriethoxysilane, 3-aminopropyltrimethoxysilane, 3-glycidoxypropyltrimethoxysilane, and isocyanatopropyltriethoxysilane, having a functional group, such as hydroxyl, carboxyl and amine, capable of covalently bonding with the dye.

More specifically, when the silica precursor (Pre_Si) is 3-aminopropyltriethoxysilane or 3-aminopropyltrimethoxysilane, a dye having a carboxyl, isothiocyanate or sulfonate functional group is used, and when the silica precursor is 3-glycidoxypropyltrimethoxysilane or isocyanatopropyltriethoxysilane, a dye having a hydroxyl or amine functional group is used.

Here, when using the dye having a carboxyl functional group, it may be immobilized to the silica precursor (Pre Si) such as 3-aminopropyltriethoxysilane or 3-aminopropyltrimethoxysilane through a carbodiimide crosslinking process using 1-ethyl-3[3-dimethyl aminopropyl]carbodiimide hydrochloride (EDC) among crosslinking agents capable of inducing amide bonding, but the kinds of crosslinking agents are not limited thereto. As for the dye having a sulfonate functional group, it may be substituted with a sulfonyl chloride group (-SO₂Cl) using a material such as POCl₃, etc. and then immobilized to a silica precursor (Pre_Si) such as 3-aminopropyltriethoxysilane or 3-aminopropyltrimethoxysilane. When the stirred reaction product of the dye and the silica precursor is formed, as shown in FIG. 2, "silica nanoparticles containing a dye immobilized inside" may be manufactured using a reverse-phase microemulsion process.

Specifically, the "silica nanoparticles containing a dye immobilized inside" may be manufactured in a manner in which the reaction product of the dye and the silica precursor and the silicate are added to a mixed solution of a surfactant and an organic solvent, stirred and added with a basic catalyst.

The surfactant is not particularly limited, but in the present invention, Triton X-100, n-hexane or the like may be used, and examples of the silicate may include, but are not limited to, tetraethyl orthosilicate, tetramethyl orthosilicate and the like.

The basic catalyst is used to promote the immobilization of the dye inside the silica nanoparticles, and may facilitate hydrolysis of the silicate due to water. The ionized silicate thus formed releases water or alcohol (ROH) through reaction and is interconnected to thus grow and form a silica network.

Examples of the basic catalyst may include ammonium hydroxide, tetrapropylammonium chloride, tetrapropylammonium hydroxide, tetrabutylammonium bromide, tetrabutylammonium chloride, tetrabutylammonium hydroxide, and the like.

In the "silica nanoparticles containing a dye immobilized inside", the dye does not leak outside, and thus stability and sensitivity may be increased, biotoxicity may be low, and the surface functional group may be easily replaced.

The diameter of the "silica nanoparticles containing a dye immobilized inside" may be 10 nm to 500 nm, and preferably 30 nm to 100 nm, within which intrinsic properties of the dye are maintained. If the diameter thereof is less than 10 nm, manipulation thereof is difficult. On the other hand, if the diameter thereof exceeds 500 nm, the optical properties of the dye may deteriorate due to the increased thickness.

FIG. 3 shows the process of synthesizing "silica nanoparticles for biomarker diagnosis containing a reactive polymer and a nonreactive polymer immobilized on the surface and a dye immobilized inside" by immobilizing a reactive polymer and a nonreactive polymer on the surface of the "silica nanoparticles containing a dye immobilized inside" according to an embodiment of the present invention.

Examples of the nonreactive polymer may include, but are not limited to, sepharose, latex, polyurethane, polyethylene, poly(ethylene glycol) silane, polyethyleneoxy poly(ethylene glycol) silane, poly(ethylene glycol) methoxysilane (or methoxy-PEG-silane), poly(ethyleneoxy) propyltriethoxysilane, 2-[methoxy(polyethyleneoxy)6-9propyl]trimethoxysilane, poly(ethylene glycol) dicarboxylic acid, hydroxy poly(ethylene glycol) carboxylic acid sepharose, poly(vinyl alcohol), polyethylene, poly(vinyl ether), polyolefin, polyester, ethylene vinyl acetate, polyamide, poly(hydroxyethyl methacrylate), poly(methyl methacrylate), polyvinylpyrrolidone, and the like.

In the present invention, examples of the reactive polymer may include not only those obtained by attaching a functional group such as a hydroxyl group, an amine group, a carboxyl group, an isocyanate group, an isothiocyanate group or an imine group to the nonreactive polymer described above, but also polyisocyanurate, polycarbamate, and the like.

The ratio of the reactive polymer and the nonreactive polymer immobilized on the surface of the silica nanoparticles for biomarker diagnosis preferably falls in the range of 1:1,000 to 1:10,000. If the ratio of the reactive polymer and the nonreactive polymer immobilized on the surface of the silica nanoparticles for biomarker diagnosis is less than 1:1,000, the dispersion stability of the silica nanoparticles for biomarker diagnosis may decrease. On the other hand, the case in which the ratio thereof exceeds 1:10,000 is not economical.

As for a biological material such as blood, a material such as a salt or protein interferes with the reaction between nanoparticles and glycated protein and also causes agglomeration of nanoparticles, thus impeding accurate measurement. Hence, when the surface of silica nanoparticles is treated with the nonreactive polymer and the reactive polymer capable of protecting nanoparticles, the effects due thereto may be minimized.

FIGS. 4 and 5 show the process of manufacturing "silica nanoparticles for biomarker diagnosis containing a reactive polymer and a nonreactive polymer immobilized on the surface and a dye immobilized inside / boronic acid", to which 3-aminophenylboronic acid (APBA) and 4-carboxylphenylboronic acid (CPBA) are bound, respectively, according to an embodiment of the present invention.

In the present invention, examples of the probe (phenylboronic acid) may include, but are not limited to, 3-aminophenylboronic acid (APBA), 4-carboxylphenylboronic acid (CPBA), etc.

When using 3-aminophenylboronic acid (APBA), a crosslinkage may be formed using disuccinimidyl carbonate as a crosslinking agent able to bind the hydroxyl residue of the reactive polymer having a hydroxyl residue and the amine residue of 3-aminophenylboronic acid (APBA) to each other, thereby immobilizing 3-aminophenylboronic acid (APBA) on the surface of the "silica nanoparticles for biomarker diagnosis containing a reactive polymer and a nonreactive polymer immobilized on the surface and a dye immobilized inside". When using 4-carboxylphenylboronic acid (CPBA), a bond may be formed through esterification between the reactive polymer having a hydroxyl residue and 4-carboxylphenylboronic acid (CPBA), thereby immobilizing 4-carboxylphenylboronic acid (CPBA) on the surface of the "silica nanoparticles for biomarker diagnosis containing a reactive polymer and a nonreactive polymer immobilized on the surface and a dye immobilized inside".

FIG. 6 shows the cis-diol bond of glycated hemoglobin and the "silica nanoparticles for biomarker diagnosis containing a reactive polymer and a nonreactive polymer immobilized on the surface and a dye immobilized inside / boronic acid" manufactured according to an embodiment of the present invention.

As shown in FIG. 6, the "silica nanoparticles for biomarker diagnosis containing a reactive polymer and a nonreactive polymer immobilized on the surface and a dye immobilized inside / boronic acid" may react with the cis-diol of glycated protein, and multiple dye molecules may be encapsulated in a silica nanoparticle, and thus the absorption of light thereof is higher than that of a single dye molecule, thereby increasing the detection sensitivity of glycated hemoglobin.

In the present invention, total protein and glycated protein bound through cis-diol to the "silica nanoparticles for biomarker diagnosis containing a reactive polymer and a nonreactive polymer immobilized on the surface and a dye immobilized inside / boronic acid" may be measured using an optical instrument such as UV/Vis or through high-performance liquid chromatography (HPLC).

Therefore, another aspect of the present invention pertains to a method of diagnosing diabetes, including measuring optical reflectance by simultaneously applying a wavelength able to measure total albumin or total hemoglobin and a wavelength able to measure glycated albumin or glycated hemoglobin bound to "silica nanoparticles for biomarker diagnosis containing a reactive polymer and a nonreactive polymer immobilized to the surface and a dye immobilized inside / boronic acid" using a light source, and then diagnosing diabetes depending on the proportion of glycated albumin or glycated hemoglobin.

In addition, still another aspect of the present invention pertains to a method of diagnosing diabetes, including reacting a biological material with "silica nanoparticles for biomarker diagnosis containing a reactive polymer and a nonreactive polymer immobilized on the surface and a dye immobilized inside / boronic acid", performing high-performance liquid chromatography (HPLC), and then diagnosing diabetes depending on the proportion of glycated albumin or glycated hemoglobin.

The proportion of glycated protein is the amount of glycated protein relative to the amount of total marker protein, and may be calculated as follows.

Proportion of glycated protein (%) = [glycated protein]/[total marker protein]

Typically, when the proportion of glycated hemoglobin is found to be 6.5% or more or the proportion of glycated albumin is found to be 20% or more (based on an enzymatic method), diabetes may be diagnosed.

### Mode for Invention

A better understanding of the present invention will be given through the following examples. These examples are merely set forth to illustrate the present invention, and are not to be construed as limiting the scope of the present invention, as will be apparent to those skilled in the art.

### Example 1: Preparation of "silica nanoparticles for biomarker diagnosis containing reactive polymer and nonreactive polymer immobilized on surface and dye immobilized inside / boronic acid" (pDSNP-PBA)

### 1-1: Preparation of stirred reaction product of dye and silica precursor

### 1-1-1: Stirred reaction product of dye having hydroxyl functional group and silica precursor (Dye-Pre_Si 1)

35 mM FD&C Green 3 was dissolved in 50 ml of a deionized (DI) water solution, added with 40 ml of a 3-glycidoxypropyltrimethoxysilane solution, and reacted at 90°C for 12 hr with stirring.

### 1-1-2: Stirred reaction product of dye having amine functional group and silica precursor (Dye-Pre _Si 2)

5 mM methylene blue was dissolved in 50 ml of a deionized water solution, added with 50 ml of an isocyanatopropyltriethoxysilane solution, and reacted at 90°C for 12 hr with stirring.

### 1-1-3: Stirred reaction product of dye having carboxyl functional group and silica precursor (Dye-Pre_Si 3)

5 mM tartrazine was dissolved in 50 ml of a 0.1 M MES (2-(N-morpholino)ethanesulfonic acid) buffer solution (pH 6.0), added with 10 mM EDC, reacted with stirring for 60 min, added with 45 ml of 3-aminopropyltriethoxysilane, and then reacted at room temperature using a stirrer for 12 hr.

### 1-2: Synthesis of silica nanoparticles containing dye immobilized inside (DSNP)

9.25 ml of the stirred reaction product solution of dye and silica precursor (Dye-Pre_Si) (1-1-1) and 2.7 ml of TEOS (tetraethyl orthosilicate) were placed in a 1 L round-bottom flask containing 150.0 ml of cyclohexane, 33.5 ml of Triton X-100 and 35.3 ml of n-hexanol, and uniformly mixed for 1 hr using a stirrer. Thereafter, 1.5 ml of 15-30% ammonia water (NH₄OH) was added thereto and reacted at room temperature for 20 hr to 30 hr, after which the reaction was terminated by the addition of 300 ml of ethanol, followed by ethanol washing four times and DI washing three times under conditions of 3900 rpm and 15 min using a centrifuge and then drying in an oven at 60°C, thus manufacturing "silica nanoparticles containing a dye immobilized inside".

### 1-3: Preparation of silica nanoparticles for biomarker diagnosis containing reactive polymer and nonreactive polymer immobilized on surface and dye immobilized inside (pDSNP)

### 1-3-1: pDSNP containing reactive polymer and nonreactive polymer immobilized at 1:1,000

100 µl of a mixed solution of (hydroxyl(polyethyleneoxy)propyl)triethoxysilane and 2-[methoxy(polyethyleneoxy)6-9propyl]trimethoxysilane at 1:1,000 was mixed with 5 ml of the "silica nanoparticles containing a dye immobilized inside" manufactured in Example 1-2 and dispersed in 99.9% ethanol, followed by stirring for 17 hr, thus manufacturing "silica nanoparticles for biomarker diagnosis containing a reactive polymer and a nonreactive polymer immobilized on the surface and a dye immobilized inside" (pDSNP).

### 1-3-2 to 1-3-5: pDSNP containing reactive polymer and nonreactive polymer immobilized at 1:100

As shown in Table 1 below, 100 µl of a mixed solution of a reactive polymer and a nonreactive polymer at 1:100 was mixed with 5 ml of the "silica nanoparticles containing a dye immobilized inside" manufactured in Example 1-2 and dispersed in ethanol, followed by stirring for 17 hr, thus manufacturing "silica nanoparticles for biomarker diagnosis containing a reactive polymer and a nonreactive polymer immobilized on the surface and a dye immobilized inside".

**[Table 1]**

| | Reactive polymer | Nonreactive polymer |
|---|---|---|
| pDSNP_ 1 | Polyethyleneimine | Polyvinyl alcohol |
| pDSNP_ 2 | Polyisocyanurate | Polyolefin |
| pDSNP_ 3 | Poly(ethylene glycol) silane carboxylic acid ((PEG-silane-COOH; Silane-PEG-COOH) | Polyamide |
| pDSNP_ 4 | (Hydroxyl (polyethyleneoxy)propyl)triethox ysilane | 2-[Methoxy(polyethyleneox y)6-9-propyl]trimethoxysilane |

### 1-4: Preparation of silica nanoparticles for biomarker diagnosis containing reactive polymer and nonreactive polymer immobilized on surface and dye immobilized inside / PBA (pDSNP-PBA) through PBA conjugation to reactive polymer immobilized on surface of nanoparticles

### 1-4-1: Preparation of silica nanoparticles for biomarker diagnosis containing reactive polymer and nonreactive polymer immobilized on surface and dye immobilized inside / 3-aminophenylboronic acid (pDSNP-APBA)

Phenylboronic acid (PBA) was conjugated to a polymer having a reactive functional group among immobilized polymers using the "silica nanoparticles for biomarker diagnosis containing a reactive polymer and a nonreactive polymer immobilized on the surface and a dye immobilized inside" manufactured in 1-3-1 as a support.

First, the hydroxyl functional group of (hydroxyl(polyethyleneoxy)propyl)triethoxysilane and the amine functional group of 3-aminophenylboronic acid (APBA) were crosslinked using disuccinimidyl carbonate, thus manufacturing "3-aminophenylboronic-acid-(APBA)-bound silica nanoparticles for biomarker diagnosis containing a reactive polymer and a nonreactive polymer immobilized on the surface and a dye immobilized inside".

Specifically, 5 ml of "silica nanoparticles for biomarker diagnosis containing a reactive polymer and a nonreactive polymer immobilized on the surface and a dye immobilized inside" dispersed in dimethyl sulfoxide (DMSO) and 100 nM disuccinimidyl carbonate dissolved in dimethyl sulfoxide (DMSO) were mixed and reacted for 2 hr. After termination of the reaction, centrifugation was performed at 11,000 rpm for 20 min, thus separating the reaction solvent and the "succinimidyl carbonate / silica nanoparticles for biomarker diagnosis containing a reactive polymer and a nonreactive polymer immobilized on the surface and a dye immobilized inside" from each other.

Next, reaction was carried out for 24 hr so as to bind 3-aminophenylboronic acid (APBA) to the "succinimidyl carbonate / silica nanoparticles for biomarker diagnosis containing a reactive polymer and a nonreactive polymer immobilized on the surface and a dye immobilized inside" (FIG. 4).

### 1-4-2: Preparation of boronic-acid-bound silica nanoparticles for biomarker diagnosis containing reactive polymer and nonreactive polymer immobilized on surface and dye immobilized inside / 4-carboxylphenylboronic acid (pDSNP-CPBA)

5 ml of "silica nanoparticles for biomarker diagnosis containing a reactive polymer and a nonreactive polymer immobilized on the surface and a dye immobilized inside" and 100 µl of 1 mg/ml 4-carboxylphenylboronic acid (CPBA) dissolved in dimethyl sulfoxide (DMSO) were stirred for 24 hr under conditions of pH 2.00, 85°C and 300 rpm. Specifically, the hydroxyl functional group of (hydroxyl(polyethyleneoxy)propyl)triethoxysilane and the carboxyl functional group of 4-carboxylphenylboronic acid (CPBA) were subjected to esterification. Through the above reaction, "4-carboxylphenylboronic-acid-bound silica nanoparticles for biomarker diagnosis containing a reactive polymer and a nonreactive polymer immobilized on the surface and a dye immobilized inside (pDSNP-CPBA)" were manufactured (FIG. 5).

### Comparative Example 1: Preparation of nanoparticles containing dye encapsulated alone (DYE SNP)

150.0 ml of cyclohexane, 33.5 ml of Triton X-100, 35.3 ml of n-hexanol, 9.25 ml of 0.035 M FD&C Green 3, and 2.7 ml of TEOS (tetraethyl orthosilicate) were placed in a 1 L round-bottom flask and uniformly mixed for 1 hr using a stirrer. Next, 1.5 ml of 25-30% ammonia water (NH₄OH) was added thereto and reacted at room temperature for 24 hr, after which the reaction was terminated by the addition of 300 ml of ethanol, followed by ethanol washing four times and DI washing three times under conditions of 3900 rpm and 15 min using a centrifuge and then drying in an oven at 60°C, thus manufacturing nanoparticles containing a dye encapsulated alone (DYE SNP).

### Test Example 1: Evaluation of difference in absorbance of "silica nanoparticles for biomarker diagnosis containing reactive polymer and nonreactive polymer immobilized on surface and dye immobilized inside / boronic acid" and nanoparticles containing dye encapsulated alone

In order to evaluate the effectiveness of the "silica nanoparticles for biomarker diagnosis containing a reactive polymer and a nonreactive polymer immobilized on the surface and a dye immobilized inside / 3-aminophenylboronic acid (pDSNP-APBA)" manufactured in Example 1-4 and the nanoparticles containing a dye encapsulated alone (DYE SNP) manufactured in Comparative Example 1, the absorption wavelengths thereof were measured using UV/Vis spectroscopy. The results thereof are shown in FIG. 7.

As shown in FIG. 7A, the nanoparticles containing a dye encapsulated alone (DYE SNP) manufactured in Comparative Example 1 did not exhibit the intrinsic absorption wavelength of FD&C Green 3, whereas the "silica nanoparticles for biomarker diagnosis containing a reactive polymer and a nonreactive polymer immobilized on the surface and a dye immobilized inside / 3-aminophenylboronic acid (pDSNP-APBA)" were observed to have a UV/Vis spectrum close to the intrinsic absorption wavelength of the dye (630 nm).

Moreover, the absorbance of the "silica nanoparticles for biomarker diagnosis containing a reactive polymer and a nonreactive polymer immobilized on the surface and a dye immobilized inside / 3-aminophenylboronic acid (pDSNP-APBA)" synthesized using Dye-Pre_Si at concentrations of 25%, 50% and 100% was measured. The results thereof are shown in FIG. 7B.

As shown in FIG. 7B, the absorbance at 630 nm was increased with an increase in the concentration of Dye-Pre_Si.

### Test Example 2: Evaluation of binding capacity of boronic acid to surface of "silica nanoparticles for biomarker diagnosis containing reactive polymer and nonreactive polymer immobilized on surface and dye immobilized inside / 3-aminophenylboronic acid (pDSNP-APBA)"

In order to evaluate whether boronic acid was bound to the surface of the "silica nanoparticles for biomarker diagnosis containing a reactive polymer and a nonreactive polymer immobilized on the surface and a dye immobilized inside / 3-aminophenylboronic acid (pDSNP-APBA)", changes in the surface residues of the "silica nanoparticles containing a dye immobilized inside (DSNP)" manufactured in Example 1-2, the "silica nanoparticles for biomarker diagnosis containing a reactive polymer and a nonreactive polymer immobilized on the surface and a dye immobilized inside (pDSNP)" manufactured in Example 1-3-1, and the "silica nanoparticles for biomarker diagnosis containing a reactive polymer and a nonreactive polymer immobilized on the surface and a dye immobilized inside / 3-aminophenylboronic acid (pDSNP-APBA)" manufactured in Example 1-4 were measured through FT-IR. The results thereof are shown in FIG. 8.

As shown in FIG. 8, silica-related bands of 1093 cm⁻¹ and 795 cm⁻¹ were observed in all of the silica nanoparticles containing a dye immobilized inside, the polymer-related peak of 3000 cm⁻¹ was observed in the "silica nanoparticles for biomarker diagnosis containing a reactive polymer and a nonreactive polymer immobilized on the surface and a dye immobilized inside (pDSNP)", and the boronic acid-related band of 1338 cm⁻¹ was additionally observed in the "silica nanoparticles for biomarker diagnosis containing a reactive polymer and a nonreactive polymer immobilized on the surface and a dye immobilized inside / 3-aminophenylboronic acid (pDSNP-APBA)". Therefore, it can be confirmed that boronic acid was immobilized through covalent bonding to the reactive polymer and the nonreactive polymer on the surface of the nanoparticles subjected to a series of synthesis processes.

### Test Example 3: Evaluation of shape and size distribution of "silica nanoparticles for biomarker diagnosis containing reactive polymer and nonreactive polymer immobilized on surface and dye immobilized inside / 3-aminophenylboronic acid (pDSNP-APBA)" and "nanoparticles containing dye encapsulated alone"

The nanoparticle size and distribution of the nanoparticles containing a dye encapsulated alone (DYE SNP) manufactured in Comparative Example 1 and the "silica nanoparticles for biomarker diagnosis containing a reactive polymer and a nonreactive polymer immobilized on the surface and a dye immobilized inside / 3-aminophenylboronic acid (pDSNP-APBA)" manufactured in Example 1-4 were evaluated using a scanning electron microscope and a dynamic light-scattering process. The results thereof are shown in FIG. 9.

As shown in FIG. 9, the particle size of the "silica nanoparticles for biomarker diagnosis containing a reactive polymer and a nonreactive polymer immobilized on the surface and a dye immobilized inside / 3-aminophenylboronic acid (pDSNP-APBA)" was increased compared to the nanoparticles containing a dye encapsulated alone (DYE SNP) under the same synthesis conditions.

### Test Example 4: Evaluation of stability of color development of "silica nanoparticles for biomarker diagnosis containing reactive polymer and nonreactive polymer immobilized on surface and dye immobilized inside / 3-aminophenylboronic acid (pDSNP-APBA)" depending on storage time after synthesis and under harsh conditions

In order to evaluate the stability of the nanoparticles containing a dye encapsulated alone (DYE SNP) manufactured in Comparative Example 1 and the "silica nanoparticles for biomarker diagnosis containing a reactive polymer and a nonreactive polymer immobilized on the surface and a dye immobilized inside / 3-aminophenylboronic acid (pDSNP-APBA)" manufactured in Example 1-4, after (A) ethanol washing 1 to 4 times, (B) heating at 100°C for 15 min, and (C) storage at room temperature for 30 days, the intrinsic absorption wavelength of the dye was measured using UV/Vis spectroscopy. The results thereof are shown in FIG. 10.

As shown in FIG. 10, the "silica nanoparticles for biomarker diagnosis containing a reactive polymer and a nonreactive polymer immobilized on the surface and a dye immobilized inside / 3-aminophenylboronic acid (pDSNP-APBA)" exhibited a UV/Vis spectrum close to the intrinsic absorption wavelength of the dye, unlike the nanoparticles containing a dye encapsulated alone (DYE SNP), and the intrinsic wavelength of the dye in the nanoparticles was maintained even upon repeated washing, heating at 100°C for 15 min or storage at room temperature for 30 days.

### Test Example 5: Evaluation of dispersion stability of "silica nanoparticles for biomarker diagnosis containing reactive polymer and nonreactive polymer immobilized on surface and dye immobilized inside (pDSNP)"

In order to evaluate the dispersion stability of the "silica nanoparticles for biomarker diagnosis containing a reactive polymer and a nonreactive polymer immobilized on the surface and a dye immobilized inside (pDSNP)", a solution including zinc chloride as a salt was reacted at different concentrations with the solution of the "silica nanoparticles for biomarker diagnosis containing a reactive polymer and a nonreactive polymer immobilized on the surface and a dye immobilized inside (pDSNP)". Here, as the silica nanoparticles for biomarker diagnosis containing a reactive polymer and a nonreactive polymer immobilized on the surface and a dye immobilized inside, silica nanoparticles obtained by immobilizing the reactive polymer and the nonreactive polymer at ratios of 1:10, 1:100, 1:500, 1:1,000 and 1:10,000 to the "silica nanoparticles containing a dye immobilized inside (DSNP)" were used.

The concentration of zinc chloride was set to 0 mM, 3 mM, 6 mM, 9 mM and 12 mM. Here, 1 ml of the silica nanoparticles for biomarker diagnosis containing a reactive polymer and a nonreactive polymer immobilized on the surface and a dye immobilized inside (pDSNP) was reacted with 100 µl of a zinc chloride solution.

As shown in FIG. 11, in the silica nanoparticles for biomarker diagnosis containing a reactive polymer and a nonreactive polymer immobilized on the surface and a dye immobilized inside (pDSNP), the dispersion stability thereof in the salt was increased with an increase in the amount of the nonreactive polymer relative to the reactive polymer. In contrast, as the amount of the nonreactive polymer relative to the reactive polymer was decreased (1:10), a great reduction in dispersion stability of the silica nanoparticles for biomarker diagnosis containing a dye immobilized inside with an increase in the salt concentration could be confirmed based on changes in the particle size with an increase in the salt concentration. Moreover, when the ratio of the reactive polymer to the nonreactive polymer was 1:1,000 and 1:10,000, changes in the particle size depending on the salt concentration were not significantly decreased, from which sufficient dispersion stability of the particles were confirmed.

Meanwhile, in order to evaluate the long-term dispersion stability depending on the ratio of the reactive polymer to the nonreactive polymer of the "silica nanoparticles for biomarker diagnosis containing a reactive polymer and a nonreactive polymer immobilized on the surface and a dye immobilized inside", among silica nanoparticles for biomarker diagnosis containing a dye immobilized inside (pDSNP), silica nanoparticles for biomarker diagnosis containing only a reactive polymer immobilized on the surface and a dye immobilized inside (pDSNP (1:0)), pDSNP (1:500), and pDSNP (1:1000), in which the ratio of the reactive polymer to the nonreactive polymer was 1:500 and 1:1000, were evaluated for dispersion stability for 4 months. The results thereof are shown in FIG. 12.

As shown in FIG. 12, among the silica nanoparticles for biomarker diagnosis containing a reactive polymer and a nonreactive polymer immobilized on the surface and a dye immobilized inside (pDSNP), the particles in which the reactive polymer and the nonreactive polymer were immobilized at ratios of 1:500 and 1:1000 agglomerated less over time than the silica nanoparticles for biomarker diagnosis containing only a reactive polymer immobilized on the surface and a dye immobilized inside (pDSNP (1:0)). The dispersion stability of the silica nanoparticles for biomarker diagnosis in which the mixed reactive and nonreactive polymers were immobilized (pDSNP (1:500, 1:1000)) was vastly superior compared to when using a single polymer alone, and the dispersion stability of the particles over time was much higher when the reactive polymer and the nonreactive polymer were immobilized at a ratio of 1:1000 than at a ratio of 1:500.

Moreover, the reduction in agglomeration of the "silica nanoparticles for biomarker diagnosis containing a reactive polymer and a nonreactive polymer immobilized on the surface and a dye immobilized inside" (pDSNP_1, pDSNP_2, pDSNP_3 and pDSNP_4) manufactured in Examples 1-3-2 to 1-3-5 depending on the salt concentration was evaluated in the same manner as above. The results thereof are shown in FIG. 13.

As shown in FIG. 13, the dispersion stability of the silica nanoparticles containing a dye immobilized inside (DSNP) was decreased with an increase in the salt concentration and thus the particle size was enlarged, whereas in the silica nanoparticles for biomarker diagnosis containing a reactive polymer and a nonreactive polymer immobilized at a ratio of 1:100 on the surface and containing a dye immobilized inside (pDSNP_1, pDSNP_2, pDSNP_3 and pDSNP_4), changes in the particle size depending on the salt concentration were not significantly decreased, from which sufficient dispersion stability of the particles were confirmed.

### Test Example 6: Evaluation of detection effectiveness of "silica nanoparticles for biomarker diagnosis containing reactive polymer and nonreactive polymer immobilized on surface and dye immobilized inside / 3-aminophenylboronic acid (pDSNP-APBA)"

In order to evaluate the effectiveness of the "silica nanoparticles for biomarker diagnosis containing a reactive polymer and a nonreactive polymer immobilized on the surface and a dye immobilized inside / 3-aminophenylboronic acid (pDSNP-APBA)", a purified glycated albumin reference sample (Sigma) was reacted at different concentrations with the "silica nanoparticles for biomarker diagnosis containing a reactive polymer and a nonreactive polymer immobilized on the surface and a dye immobilized inside / 3-aminophenylboronic acid (pDSNP-APBA)", after which the concentration of glycated albumin bound to the nanoparticles was measured. 5 µl of glycated albumin at various concentrations (0, 10, 30 and 40 mg/ml) and 0.2 ml of "silica nanoparticles for biomarker diagnosis containing a reactive polymer and a nonreactive polymer immobilized on the surface and a dye immobilized inside / 3-aminophenylboronic acid (pDSNP-APBA)" (50 mM HEPES, 8.1) were reacted at room temperature for 1 hr with stirring, after which unbound glycated albumin was removed through centrifugation and washing with a 50 mM HEPES buffer.

The glycated albumin bound to the "silica nanoparticles for biomarker diagnosis containing a reactive polymer and a nonreactive polymer immobilized on the surface and a dye immobilized inside / 3-aminophenylboronic acid (pDSNP-APBA)" was recovered and measured using an optical spectrometer at the intrinsic wavelength of the dye (630 nm) and the protein quantitative measurement wavelength (540 nm). The results thereof are shown in FIG. 14.

As shown in FIG. 14, the amount of glycated albumin (GA) bound to the "silica nanoparticles for biomarker diagnosis containing a reactive polymer and a nonreactive polymer immobilized on the surface and a dye immobilized inside / 3-aminophenylboronic acid (pDSNP-APBA)" was increased in proportion to the concentration thereof.

Although specific embodiments of the present invention have been disclosed in detail as described above, it will be obvious to those skilled in the art that the description is merely of preferable exemplary embodiments and is not to be construed to limit the scope of the present invention. Therefore, the substantial scope of the present invention will be defined by the appended claims and equivalents thereof.

### Industrial Applicability

According to the present invention, silica nanoparticles for biomarker diagnosis are configured such that a nonreactive polymer for increasing dispersion stability and a probe-bound reactive polymer are immobilized on the surface of silica nanoparticles containing a dye immobilized inside, thus minimizing the influence of various proteins and salts contained in samples, and can be applied to a point-of-care testing system using an actual biological material to thus quickly and accurately measure a biomarker to be detected in a stable manner.

## Claims

1. A silica nanoparticle for biomarker diagnosis containing a reactive polymer and a nonreactive polymer immobilized on a surface and a dye immobilized inside.

2. The silica nanoparticle of claim 1, wherein the biomarker is a blood protein comprising glycated hemoglobin or glycated albumin.

3. The silica nanoparticle of claim 1, wherein the dye is a dye having at least one functional group selected from the group consisting of a hydroxyl functional group, a carboxyl functional group, an amine functional group, an isothiocyanate functional group, and a sulfonate functional group.

4. The silica nanoparticle of claim 3, wherein the dye having a hydroxyl functional group is at least one selected from the group consisting of FD&C Green 3, calcein blue, calcein blue AM, Carmine, Coelenterazine, Coelenterazine cp, Coelenterazine f, Coelenterazine h, Coelenterazine hcp, Coelenterazine n, CoroNa Green, Hematoxylin, Hoechst 33258, Newport green pdx, Oil red O, Orange II, Orcein, Oxonol V, Oxonol VI, Ponceau SX, Sudan III, Sudan IV, and Sunset Yellow FCF.

5. The silica nanoparticle of claim 3, wherein the dye having a carboxyl functional group is at least one selected from the group consisting of tartrazine, betanin, bixin, carminic acid, galiosin, pseudo-purpurin, BAPTA, BTC (benzene-1,3,5-tricarboxylic acid), calcein, calcium green 1, calcium green 2, calcium green 5N, Eosin B, Eosin Y, erythrosine, Fluo-3, Fluo-4, FluoZin-2, FluoZin-3, Fura-2, Fura-2FF, Indo 1, PBFI (potassium-binding benzofuran isophthalate), RhodZin-3, SBFI (sodium benzofuran isophthalate), and Zinquin.

6. The silica nanoparticle of claim 3, wherein the dye having a primary amine functional group is at least one selected from the group consisting of ACMA (9-amino-6-chloro-2-methoxyacridine), methylene blue, Nile blue, toluidine blue O, Azure A, Azure B, basic fuchsin, Bismarck brown Y, brilliant cresyl blue, cresyl violet acetate, DAF-FM (diaminofluorescein-FM), dansylcadaverine, DAPI (4',6-diamidino-2-phenylindole), dihydroethidium, dihydrorhodamine 123, ethidium bromide, ethidium homodimer-1, ethidium homodimer-2, ethidium monoazide, hexidium iodide, Lucifer yellow CH, Lucifer yellow VS, neutral red, nuclear yellow, pararosaniline hydrochloride, propidium iodide, rhodamine 123, Safranin O and thionine, and the dye having a secondary amine functional group is Auramine O.

7. The silica nanoparticle of claim 3, wherein the dye having an isothiocyanate functional group is at least one selected from the group consisting of fluorescein isothiocyanate, malachite green isothiocyanate, rhodamine B isothiocyanate, and stilbene isothiocyanate sulfonic acid.

8. The silica nanoparticle of claim 3, wherein the dye having a sulfonate functional group is at least one selected from the group consisting of xylene cyanol FF, Alizarin red S, Allura red, amaranth, aniline blue, brilliant blue FCF, Congo red, Indigo carmine, Light Green SF Yellowish, methyl orange, Orange G, SPQ (6-methoxy-N-(3-sulfopropyl)quinolinium), and XTT (2,3-bis-(2-methoxy-4-nitro-5-sulfophenyl)-2H-tetrazolium-5-carboxanilide) .

9. The silica nanoparticle of claim 1, wherein the "silica nanoparticle containing a dye immobilized inside" is manufactured in a manner in which a stirred reaction product of a dye and a silica precursor and silicate are added to a mixed solution of a surfactant and an organic solvent, stirred, and added with a basic catalyst.

10. The silica nanoparticle of claim 1, wherein the nonreactive polymer is at least one selected from the group consisting of sepharose, latex, polyurethane, polyethylene, poly(ethylene glycol) silane, polyethyleneoxy poly(ethylene glycol) silane, poly(ethylene glycol) methoxysilane, poly(ethyleneoxy) propyltriethoxysilane, 2-[methoxy(polyethyleneoxy)6-9propyl]trimethoxysilane, poly(ethylene glycol) dicarboxylic acid, hydroxy poly(ethylene glycol) carboxylic acid sepharose, poly(vinyl alcohol), polyethylene, poly(vinyl ether), polyolefin, polyester, ethylene vinyl acetate, polyamide, poly(hydroxyethyl methacrylate), poly(methyl methacrylate), and polyvinylpyrrolidone.

11. The silica nanoparticle of claim 1, wherein the reactive polymer is configured such that a functional group selected from the group consisting of a hydroxyl group, an amine group, a carboxyl group, an isocyanate group, an isothiocyanate group and an imine group is bound to the nonreactive polymer of claim 10.

12. The silica nanoparticle of claim 1, wherein the reactive polymer is at least one selected from the group consisting of polyisocyanurate and polycarbamate.

13. The silica nanoparticle of claim 1, wherein the silica nanoparticle has a diameter of 10 nm to 500 nm.

14. A method of manufacturing a silica nanoparticle for biomarker diagnosis containing a reactive polymer and a nonreactive polymer immobilized on a surface and a dye immobilized inside, comprising:
(a) reacting a dye with a silica precursor;
(b) manufacturing a "silica nanoparticle containing a dye immobilized inside" by adding a reaction product of the dye and the silica precursor and silicate to a mixed solution of a surfactant and an organic solvent, performing stirring and then adding a basic catalyst thereto;
(c) immobilizing a "reactive polymer" and a "nonreactive polymer" on a surface of the "silica nanoparticle containing a dye immobilized inside" by stirring the "reactive polymer" and the "nonreactive polymer" with the "silica nanoparticle containing a dye immobilized inside"; and
(d) binding a predetermined probe to the "reactive polymer".

15. The method of claim 14, wherein the silica precursor is selected from the group consisting of 3-aminopropyltriethoxysilane, 3-aminopropyltrimethoxysilane, 3-glycidoxypropyltrimethoxysilane, and isocyanatopropyltriethoxysilane.

16. The method of claim 14, wherein the probe is 3-aminophenylboronic acid (APBA) or 4-carboxylphenylboronic acid (CPBA).

17. A method of diagnosing diabetes, comprising:
measuring optical reflectance by simultaneously applying a wavelength able to measure total albumin or total hemoglobin and a wavelength able to measure glycated albumin or glycated hemoglobin bound to a "silica nanoparticle for biomarker diagnosis containing a reactive polymer and a nonreactive polymer immobilized on a surface and a dye immobilized inside / boronic acid" using a light source, and then diagnosing diabetes depending on a proportion of glycated albumin or glycated hemoglobin.

18. A method of diagnosing diabetes, comprising:
reacting a biological material with a "silica nanoparticle for biomarker diagnosis containing a reactive polymer and a nonreactive polymer immobilized on a surface and a dye immobilized inside / boronic acid", performing high-performance liquid chromatography (HPLC), and then diagnosing diabetes depending on a proportion of glycated albumin or glycated hemoglobin.
